Europäisches Patentamt

European Patent Office

Office européen des brevets

(19)

(11) **EP 0 956 054 B1**

(12) **EUROPÄISCHE PATENTSCHRIFT**

(45) Veröffentlichungstag und Bekanntmachung des Hinweises auf die Patenterteilung:
**24.04.2002 Patentblatt 2002/17**

(51) Int Cl.[7]: **A61L 2/00**

(21) Anmeldenummer: **98904076.1**

(86) Internationale Anmeldenummer:
**PCT/EP98/00206**

(22) Anmeldetag: **15.01.1998**

(87) Internationale Veröffentlichungsnummer:
**WO 98/34649 (13.08.1998 Gazette 1998/32)**

(54) **VERFAHREN ZUM VERDAMPFEN UND ÜBERHITZEN EINES STERILISIERUNGSMITTELS UND VORRICHTUNG HIERFÜR**

METHOD FOR VAPORIZING AND SUPERHEATING A STERILIZING AGENT AND DEVICE THEREFOR

PROCEDE ET DISPOSITIF POUR VAPORISER ET SURCHAUFFER UN AGENT DE STERILISATION

(84) Benannte Vertragsstaaten:
**BE DE ES FR IT PT**

(30) Priorität: **07.02.1997 DE 19704639**

(43) Veröffentlichungstag der Anmeldung:
**17.11.1999 Patentblatt 1999/46**

(73) Patentinhaber: **Tetra Laval Holdings & Finance SA 1009 Pully (CH)**

(72) Erfinder: **SÖRENSEN, Karsten D-64347 Griesheim (DE)**

(74) Vertreter: **Weber, Dieter, Dr. et al Weber, Dieter, Dr., Seiffert, Klaus, Dipl.-Phys., Lieke, Winfried, Dr., Gustav-Freytag-Strasse 25 65189 Wiesbaden (DE)**

(56) Entgegenhaltungen:
**EP-A- 0 427 051        FR-A- 2 609 149 US-A- 4 896 478**

**Beschreibung**

Die Erfindung betrifft ein Verfahren zum Verdampfen und Überhitzen eines Sterilisierungsmittels, bei welchem das Sterilisierungsmittel mit Hilfe einer warmen Oberfläche auf eine erste Temperatur gebracht und verdampft und der Dampf nachfolgende durch Heizelemente auf eine zweite, höhere Temperatur gebracht und überhitzt wird.

Die Erfindung betrifft auch eine Vorrichtung dieser Art, die eine Verdampfungskammer und einen dieser nachgeschalteten Heizkörper mit Überhitzungskanälen und Heizelementen aufweist.

Es ist bekannt, Flüssigkeiten und insbesondere Wasser zu vielerlei Zwecken zu verdampfen und zu überhitzen. Dabei wird in einem ersten Behälter oder Kessel feuchter Dampf durch Erhitzen der Flüssigkeit erzeugt. Der Dampf wird danach in einen zweiten Behälter oder Kessel geleitet, der ohne Druckerhöhung den Dampf überhitzt, d.h. über die Siedetemperatur der Flüssigkeit erwärmt, so daß der aus diesem Kessel austretende Dampf überhitzt ist und als trockener Dampf bezeichnet werden kann.

Es gibt verschiedene wasserähnliche Flüssigkeiten, die man bei Bedarf solchen Verfahren unterziehen kann. Zum Beispiel ist es biswellen erwünscht, Sterilisierungsmittel zu überhitzen, um diese als überhitzten Dampf auf zu sterilisierende Flächen aufzubringen. Die bekannten Verfahren und Vorrichtungen zur Erzeugung von überhitzten Sterllisierungsdämpfen haben den Nachteil, daß sie aufwendig sind, eine verhältnismäßig große Wärmemenge benötigen und nur in Vorrichtungen erzeugt werden können, die einen großen Platzbedarf haben.

Der Erfindung liegt daher die Aufgabe zugrunde, die bekannten Verfahren zu vereinfachen und effektiver zu gestalten und für die Vorrichtung technisch einfachere und kompaktere Bauelemente zu verwenden.

Für das Verfahren gelingt die Lösung der Aufgabe gemäß der Erfindung dadurch, daß

a) das Sterilisierungsmittel auf die warme Oberfläche aufgesprüht wird, die auf eine erste Temperatur erwärmt wird,
b) welche kleiner ist als die Oberflächentemperatur, bei welcher das Filmsleden beginnt,
c) die erste Temperatur der warmen Oberfläche abgefühlt und in Signale zur Regelung der Heizelemente für das Überhitzen umgewandelt wird, und
d) der Dampf des Sterilisierungsmittels in Gegenstrom derart erwärmt wird, daß die erste Temperatur durch die zweite höhere Temperatur und durch einen dem Dampfstrom entgegen gerichteten Wärmefluß im wesentlichen konstant gehalten wird.

Während bei den bekannten Verfahren und Vorrichtungen zur Erzeugung von überhitzten Sterilisierungsmitteln die warme Oberfläche sozusagen die Innenoberfläche des Behälters ist, In welchem die Flüssigkeit zum Sieden gebracht wird, oder in einem Kessel auch die Innenoberfläche von Rohren ist, auf bzw. in welcher die zu erwärmende und zu verdampfende Flüssigkeit liegt, wird erfindungsgemäß das Sterilisierungsmittel durch Aufsprühen mit der warmen Oberfläche in Berührung gebracht. Es kommt also nicht der massive Flüssigkeitskörper mit der warmen Oberfläche in Berührung, sondern nur ein Nebel des Sterilisierungsmittels, also eine große Anzahl fein verteilter Tröpfchen. Dadurch werden die Wirkoberfläche des flüssigen Sterilsierungsmittels erheblich vergrößert und der Wärmeübergang von der wannen Oberfläche auf das jeweilige Flüssigkeitströpfchen verbessert.

Wenn nun die erwähnte erste Temperatur dieser warmen Oberfläche kleiner ist als die Oberflächentemperatur, bei der das Filmsieden des betreffenden Sterilisierungsmittels beginnt, dann wird aus nachfolgend noch zu erklärenden Gründen die gesamte Wärmebilanz besonders günstig. Man benötigt also für die Verdampfung und Überhitzung des Sterilisierungsmittels nach dem erfindungsgemäßen Verfahren eine erheblich geringere Wärmemenge und kann dadurch Energie sparen. Dadurch, daß man die Temperatur der sogenannten warmen Oberfläche in dem erwähnten Bereich hält, gewährleistet man einen hohen Wärmegradienten und damit einen großen Wärmefluß von der bewußt im Abstand von der warmen Oberfläche angeordneten Heizung zu der warmen Oberfläche hin.

Diese Heizung kann günstig gesteuert und zur Regelung des erfindungsgemäßen Verfahrens verwendet werden, wenn ein Temperaturfühler entsprechend der ersten Temperatur der warmen Oberfläche Signale erzeugt, mit denen die Heizelemente für das Überhitzen geregelt werden. Es wird durch die Heizelemente mit anderen Worten stets eine solche Wärmemenge erzeugt, daß sie zu der warmen Oberfläche strömt und diese auf der vorstehend erwähnten Temperatur hält.

An der warmen Oberfläche werden die vielen kleinen Tröpfchen des zunächst flüssigen Sterilisierungsmittels verdampft, und dieser Dampf strömt, durch Druckunterschiede beeinflußt, in eine Richtung, welche der Richtung des Wärmeflusses entgegenläuft. Auf diese Weise wird der Dampf des Sterilisierungsmittels durch das neue Verfahren im Gegenstrom erwärmt. Mit Vorteil wird die erste Temperatur der warmen Oberfläche durch eine zweite höhere Temperatur im abstromigen Bereich des Verfahrens auf dem richtigen Niveau gehalten, weil eben der dem Dampfstrom entgegengerichtete Wärmefluß erzeugt wird. Dadurch kann man die warme Oberfläche auf der gewünschten ersten Temperatur halten.

Durch das erfindungsgemäße Verfahren erreicht man für die Verdampfung der Nebeltröpfchen des Sterilisierungsmittals die optimale Temperatur der warmen Oberfläche. Es ist nämlich sehr bedeutsam, die richtige Temperatur der warmen Oberfläche zu finden, einzustellen und im Betrieb auf dem richtigen Wert zu halten. Aus einem Diagramm, das nachfolgend noch erläutert wird, ergibt sich theoretisch, daß bei der optimalen Temperatur der warmen Oberfläche eine verhältnismäßig große Wärmemenge von der warmen Oberfläche auf die Nebeltröpfchen des Sterilisierungsmittels übertragen werden kann. Im Betrieb der Anmelderin hat man vorher schon andere Versuche mit anderen Heizmethoden durchgeführt und zum Beispiel eine Verdampfung und Überhitzung mittels heißer Luft unterschiedlicher Temperaturen versucht. Der Wirkungsgrad solcher Verfahren bleibt aber weit hinter dem nach dem erfindungsgemäßen Verfahren zurück.

Erreicht man nun durch Befolgung der erfindungsgemäßen Lehre eine große Wärmeabfuhr an der aufstromigen warmen Oberfläche, dann können große Mengen flüssigen Sterilisierungsmittels pro Zeiteinheit verdampft werden. Bei richtiger Einstellung der Heizelemente, d.h. bei richtiger Temperatur des überhitzten Dampfes im abstromigen Bereich des Verfahrens, erhält man einen erheblich kleineren Wärmefluß in den Dampf hinein, so daß die durch die Heizelemente erzeugte Wärmemenge im Gegenstrom zum fließenden Dampf zu der warmen Oberfläche strömt. Dadurch läßt sich auf sehr einfache Weise die optimale Temperatur dieser warmen Oberfläche einstellen und halten.

Wenn bei weiterer vorteilhafter Ausgestaltung der Erfindung das Sterilisierungsmittel Wasserstoffperoxid ist, läßt sich das Verdampfungsverfahren unter wasserähnlichen Bedingungen mit guter Wirkung einsetzen. Darunter versteht man, daß Wasserstoffperoxid mit unterschiedlichen Konzentrationen verwendet werden kann. Wasserstoffperoxid hat bezüglich der Temperatur-, Verdampfungs- und Überhitzungseigenschaften ähnliche Merkmale wie Wasser.

Es hat sich für die Durchführung des erfindungsgemäßen Verfahrens für günstig erwiesen, wenn man bei einer bevorzugten Ausführungsform die erste Temperatur der warmen Oberfläche im Bereich von 100°C bis 150°C, vorzugsweise im Bereich von 120°C bis 140°C hält.

Trägt man in einem Diagramm über einer Temperaturdifferenz, welche durch die aktuelle Temperatur minus der Siedetemperatur des Sterilisierungsmittels gebildet wird, gemessen in Grad Celsius, die von der Oberfläche des Sterilisierungsmittels aufnehmbare Wärmemenge, In Watt pro Quadratmeter gemessen, auf, dann findet sich für Wasser ein erstes Maximum bei etwa 130°C aktueller Temperatur. Bei dieser Temperatur kann also die maximale Wärmemenge von der warmen Oberfläche auf das Sterilisierungsmittel übertragen werden.

Ganz anders liegen die Verhältnisse bei Temperaturen von zum Beispiel 200°C. Ist der Dampf des Sterilisierungsmittels durch das erfindungsgemäße Verfahren, d.h. durch die Wirkung der Heizelemente auf eine solche Temperatur überhitzt worden, dann zeigt das Diagramm eine stark gesunkene Wärmemenge, welche auf das Sterilisierungsmittel übertragen werden kann.

Die von den Heizelementen erzeugte Wärmemenge kann aber durch den hohen Wärmeverbrauch der warmen Oberfläche zu letzterer strömen, weshalb sich der vorstehend erwähnte Wärmefluß ergibt.

Es kann günstig sein, eine andere und zusätzliche Maßnahme erfindungsgemäß bei dem neuen Verfahren anzuwenden, welche erfindungsgemäß dadurch gekennzeichnet ist, daß ein gasförmiges Trägermedium in den Sprühstrahl des Sterilisierungsmittels eingeführt wird. Ein solches Trägermedium könnte zum Beispiel Luft oder ein anderes gasförmiges Medium sein. Dadurch kann der Verbrauch an Sterilisierungsmittel verringert werden, wenn zum Beispiel die Konzentration des letztlich anzuwendenden Sterilisierungsmittels nicht 100%, 80% oder dergleichen zu sein braucht. Man kann das Trägermedium gegebenenfalls auch als Treibmittel verwenden, wenn man es mit entsprechender Strömungsgeschwindigkeit einführt, zum Beispiel in den Sprühstrahl des Sterilisierungsmittels einbläst.

Vorteilhaft ist es gemäß der Erfindung ferner, wenn das gasförmige Trägermedium eine Temperatur im Bereich von 130°C bis 170°C, vorzugsweise von 120°C bis 160°C und besonders bevorzugt von etwa 150°C hat. Bei der Verwendung dieser Temperaturen für das Trägermedium erreicht man gute Wirkungsgrade für Wasserstoffperoxid als Sterillsierungsmittel. Es wird einerseits dessen Verdünnung undloder gegebenenfalls dessen Antrieb erreicht und durch die Temperatur des Trägermediums verhindert, daß dieses dem Sterilisierungsmittel nicht Wärme wegnimmt.

Die vorstehend genannte Aufgabe wird bei der Vorrichtung erfindungsgemäß dadurch gelöst, daß

a) die warme Oberfläche die aufstromige Oberfläche des Heizkörpers ist,
b) an welcher die Verdampfungskammer direkt angebaut ist,
c) in der Verdampfungskammer gegenüber der warmen Oberfläche eine mit einer nach außerhalb führenden Zuleitung verbundene Düse angebracht ist,
d) von der aufstromigen Oberfläche zur abstromigen Endfläche des Heizkörpers offene, durchlaufende Kanäle vorgesehen sind und daß
e) die Heizelemente im abstromigen Endbereich des Heizkörpers untergebracht sind, und
f) ein Temperaturfühler ein der ersten Temperatur entsprechendes Signal erzeugt, mit dem die Heizelemente geregelt werden.

Bei einer derart aufgebauten Verdampfungsvorrichtung gelingt auf engem Raum und mit einfachen Mitteln die Überhitzung des verdampften Mediums, zum Beispiel eines Sterilisierungsmittels. Durch die Anordnung der Sprühdüse gegenüber und aufstromig von der warmen Oberfläche wird die zu verdampfende Flüssigkeit, zum Beispiel das Sterilisierungsmittel, in einen Nebel feinster Tröpfchen verteilt. Dadurch, daß die Verdampfungskammer direkt an der aufstromigen Oberfläche des Heizkörper angebaut ist, können die feinen Tröpfchen des Sterilisierungsmittels mit der warmen Oberfläche in Kontakt kommen und die für die Verdampfung erforderliche Energie aufnehmen. Der Betrieb kann kontinuierlich erfolgen, denn das Sterilisierungsmittel kann von der Zuleitung außerhalb der Verdampfungskammer fortlaufend der Düse zugeführt werden, wo das Sterilisierungsmittel vernebelt wird.

Wenn nach diesen Maßnahmen Dampf in der Verdampfungskammer vorliegt, der infolge vorgegebener Druckdifferenzen sich weiter abstromig bewegen will, gelingt die Überhitzung, direkt hinter der warmen Oberfläche des Heizkörpers beginnend, in einfacher Weise dadurch, daß der Dampf durch die durch den Heizkörper vollständig hindurchlaufenden Kanäle strömt und während dieses Strömens von den warmen (beheizten) Wänden dieser Kanäle Wärmeenergie aufnimmt Diese weitere Wärmeaufnahme zur Überhitzung des Dampfes erfolgt hinter der aufstromigen Oberfläche des Heizkörpers, welche die sogenannte warme Oberfläche ist, bis zu der abstromigen Endfläche des Heizkörpers, in welcher die Kanäle letztlich münden. Dies ist die abstromige Seite des Heizkörper, in deren Nähe die Heizelemente angeordnet sind. Dadurch ist gewährleistet, daß die höchste Temperatur am abstromigen Ende des Heizkörpers vorliegt und der Heizkörper In diesem abstromigen Endbereich die größte Wärmemenge enthält, die zum aufstromigen Ende abzuwandern beginnt

Dieser Wärmefluß vom abstromigen Endbereich zur aufstromigen Oberfläche des Heizkörpers sorgt einerseits dafür, daß die gewünschte Temperatur an der warmen Oberfläche erreicht und aufrechterhalten wird und sorgt andererseits für einen intensiven Wärmetransport, der notwendig ist, weil von der warmen Oberfläche etwa die zehnfache Wärmemenge an die zu verdampfende Flüssigkeit (die Tröpfchen) abgegeben wird als in den Kanälen im abstromigen Endbereich.

Geht man von dem Beispiel Wasser aus, welches an der warmen Oberfläche bei 100°C von der flüssigen in die Dampfphase überführt werden soll, dann ist eine Wärmemenge von 2257 kJ/kg erforderlich.

Will man Dampf im abstromigen Endbereich zum Überhitzen erwärmen, dann braucht man dagegen für dieses Überhitzen pro Grad Celsius nur

$$2 \text{ kJ/kg} \times \text{K},$$

wobei K bedeutet: Grad Kelvin.

Wünscht man den Dampf bei 100°C an der warmen Oberfläche für ein gutes Überhitzen auf etwa 200°C im abstromigen Endbereich zu erwärmen, dann bedeutet dies eine Temperaturdifferenz von 100°C. Infolgedessen ist die erforderlich Wärmemenge für 100°C das Hundertfache von dem vorstehend angegebenen Betrag. Es handelt sich also nur um 200 kJ/kg, also ein Zehntel der bei der Verdampfung erforderlichen Wärmemenge. Dadurch kann sich im abstromigen Endbereich eine hohe Temperatur im Betrieb aufbauen und sich auch während des Betriobes des Überhitzens halten.

Wollte man eine noch höhere Temperatur durch das Überhitzen im abstromigen Endbereich erreichen, dann brauchte man den Heizkörper nur entsprechend zu verlängern. Bei dem vorstehenden Beispiel mit einer Überhitzungstemperatur im abstromigen Endbereich von etwa 200°C genügt aber ein verhältnismäßig kurzer, kompakter Heizkörper.

Ein betriebliches Beispiel für eine gute Verdampfung und Überhitzung eines Sterilisierungsmittels wurde mit einem Heizkörper mit einem Durchmesser von etwa 150 mm und einer Länge von etwa 230 mm erreicht.

Zweckmäßig ist es gemäß der Erfindung femer, wenn die Verdampfungskammer mit einer Pumpe oder einem Gebläse für gasförmiges Trägermaterial verbunden ist und sich zur warmen Oberfläche hin erweitert. Zum Beispiel kann man Heißluft mit einer Temperatur von etwa 150°C als ein solches gasförmiges Trägermaterial verwenden. Gase und Nebel verteilen sich durch das zugepumpte Trägermaterial und kommen mit der gesamten warmen Oberfläche in intensive Berührung. Daraus ergibt sich ein guter Wärmeübergang und eine gute Wärmeabfuhr, um ein schnelles und intensives Verdampfen zu erreichen. Dies gelingt auch dann, wenn von der warmen Oberfläche die Wärmekanäle abgehen, so daß die Oberfläche dadurch etwas verringert wird.

Es ist günstig, wenn man den Heizkörper aus einem Material mit gutem Wärmeleitungskoeffizienten ausbildet. Hier hat sich Metall als günstig erwiesen, insbesondere Aluminium.

Man kann erfindungsgemäß ferner den Heizkörper außen auch mit einer wärmeisolierenden Schicht versehen. Der Temperaturgradient vom abstromigen Endbereich zur aufstromigen warmen Oberfläche ändert sich dadurch aber erstaunlicherweise wenig.

Bei vorteilhafter weiterer Ausgestaltung der Erfindung ist der Heizkörper ein massiver, zylinderförmiger Metallblock, die aufstromige Oberfläche und die abstromige Endfläche sind eben und mit Löchern versehen, an welche sich gerade, parallele Kanäle anschließen, und die aufstromig angebrachte Verdampfungskammer sowie der abstromig angebrachte Sammeltrichter sind zu den Ebenen des Heizkörpers als sich erweiternd ausgestaltete Trichter ausgebildet. Dadurch kann man ein durch eine Leitung mit kleinem Durchmesser zugeführtes Transportmittel mit einem seitlich von außerhalb durch eine Zuleitung zugeführten anderen Mittel, zum Beispiel dem Sterilisierungsmittel, vermischen und auf die gesamte warme Oberfläche aufbringen, weil das Gemisch durch die trichterförmige Erweiterung auch auf die äußeren Bereiche der warmen Oberfläche geführt wird. Wenn umgekehrt hinter der abstromigen Endfläche wiederum ein Sammeltrichter an-

gebracht ist, der sich von der Endfläche In Abstromrichtung verjüngt, dann kann man alle Dampfströmungen aus den Kanälen bündeln und in einer Ableitung sammeln. Nach einer Durchströmung von einer Wegstrecke von etwa 1 m ist die Sammelflasche des überhitzten Sterilisierungsmittels zu denken. Die Überhitzungstemperatur braucht jetzt nur so hoch eingestellt zu werden, daß das überhitzte Sterilisierungsmittel oder das Gemisch aus Luft mit überhitztem Sterilisierungsmittel seine Phase und möglichst auch seine Temperatur auf dem Weg bis zu Lagerflasche nicht wesentlich verändert.

Gute Ergebnisse erreicht man bei im wesentlichen gleicher Strömungsgeschwindigkeit des In Überhitzung befindlichen Dampfes durch die erwähnten Kanäle. Wünscht man Indessen eine fünffache Dampfmenge pro Zeiteinheit, dann wird die Anordnung von fünfmal so viel Kanälen verwendet Mit anderen Werten hängt die Anzahl der Kanäle von dem durchströmenden Dampfvolumen ab.

Weitere Vorteile, Merkmale und Anwendungsmöglichkeiten der vorliegenden Erfindung ergeben sich aus der folgenden Beschreibung bevorzugter Ausführungsformen in Verbindung mit den anliegenden Zeichnungen. Bei diesen zeigen:

Figur 1 die vergrößerte Querschnittsansicht eines schematisch in Figur 2 gezeigten Heizkörpers,

Figur 2 isometrisch eine vereinfachte und schematische Darstellung eines Heizkörpers mit der aufstromig angeordneten Verdampfungskammer und dem abstromig angebrachten Sammeltrichter,

Figur 3 eine praktische Ausführungsform ohne die Flasche unten und die Zuführelemente oben,

Figur 4 vergrößert und isometrisch die Darstellung einer anderen praktischen Ausführungsform eines Heizkörpers und

Figur 5 ein Diagramm unter Darstellung der Siedekurve für Wasser bei einer Atmosphäre.

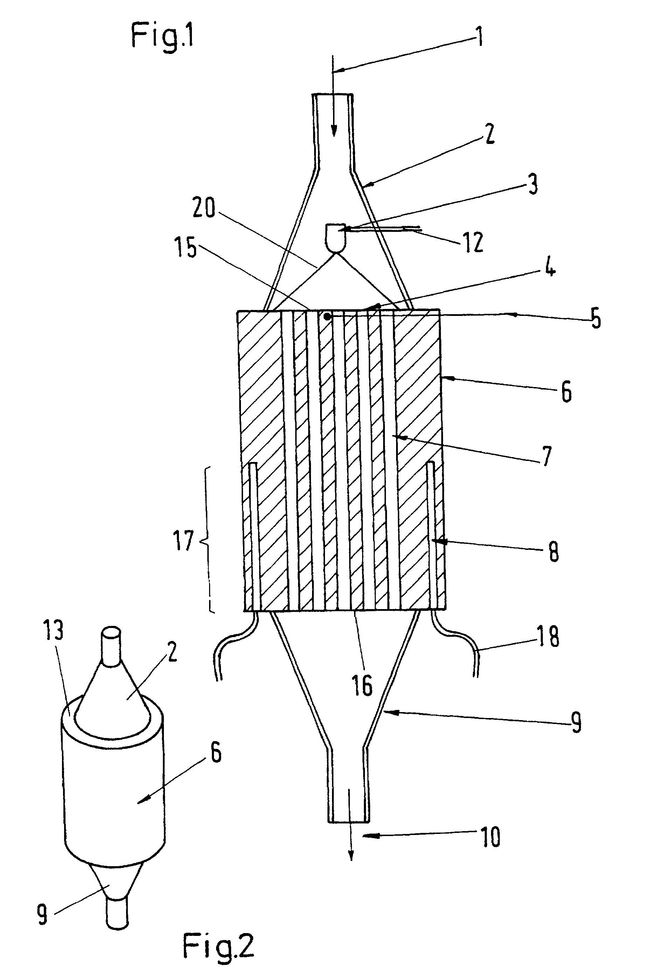

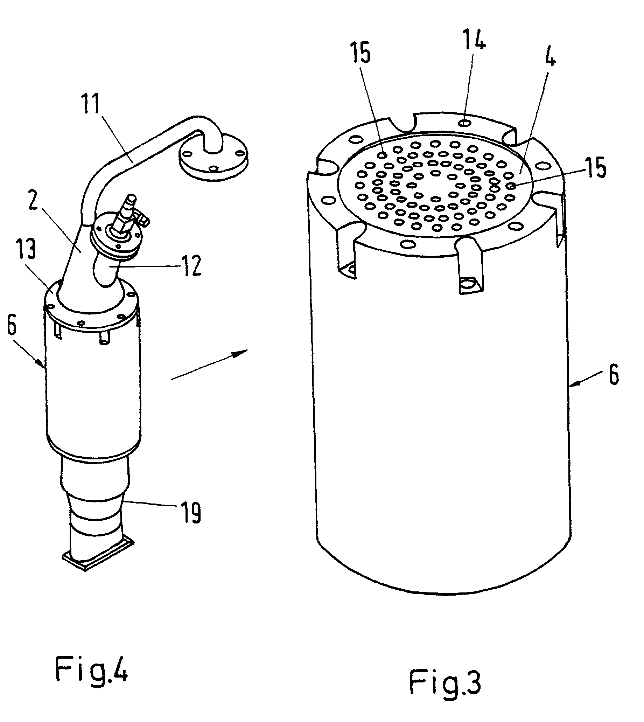

Bei der vereinfachten Ausführungsform der Figur 1 wird Heißluft in Richtung des Pfeiles 1 mit Hilfe nicht dargestellter Pumpen über die in Figur 4 gezeigte Zuleitung 11 in die Verdampfungskammer 2 geführt. Die Verdampfungskammer sitzt wie ein umgekehrter Trichter auf der ebenen, warmen Oberfläche 4, deren Temperatur über einen bei 5 angedeuteten Meßfühler erfaßt wird. Gegenüber der warmen Oberfläche 4 befindet sich eine Düse 3 etwa in der Mitte der trichterförmigen Verdampfungskammer 2 und ist mit einer nach außerhalb der Verdampfungskammer 2 führenden Zuleitung 12 verbunden.

In Figur 4 erkennt man, daß die tatsächliche Anordnung der Zuführleitung 12 für das Zuführen von flüssigem Wasserstoffperoxid als Sterilisierungsmittel anders ausgestaltet und unter einem anderen Winkel zu der hier vertikal angenommenen Strömungsrichtung 1 ausgebildet und angestellt ist.

Über den aufstromigen Flansch 13 (Figur 4) ist die Verdampfungskammer 2 an dem allgemein mit 6 bezeichneten Heizkörper angeschraubt, der oben Schraublöcher 14 für die Befestigung des Flansches 13 trägt. Die aufstromige ebene Fläche, d.h. die sogenannte warme Oberfläche 4 des Heizkörpers 6 ist in Figur 3 in schräger Draufsicht als ebene Scheibe mit einer Vielzahl von Löchern 15 zu sehen.

Unter diesen auch in Figur 1 angedeuteten Löchern 15 schließen sich gerade, parallel zueinander verlaufende Kanäle 7 an. Sie befinden sich im Abstand voneinander, wie man in der Schnittdarstellung des Heizkörpers 6 in Figur 1 sieht, und enden in der abstromigen Endfläche 16 des Heizkörpers 6. Im abstromigen Endbereich 17 des Heizkörpers 6 sind kranzartig außen Heizkörper 8 eingelassen, bei denen es sich bei der hier gezeigten Ausführungsform um elektrische Heizstäbe handelt, deren elektrische Anschlußkabel bei 18 angedeutet sind.

Unten schließt sich an die abstromige Endfläche 16, die ebenfalls eine ebene Scheibe mit Löchern ist, der Sammeltrichter 9 an, der sich kegelstumpfförmig im Gegenstrom des überhitzten Dampfes zur abstromigen Endfläche 16 hin erweitert und daher die strömenden, überhitzten einzelnen Dampfstrahlen vereinigt und in Richtung des Pfeiles 10 durch die Ableitung 19 (Figur 4) zu einer unten angeschlossenen, nicht dargestellten Sammelflasche abführt.

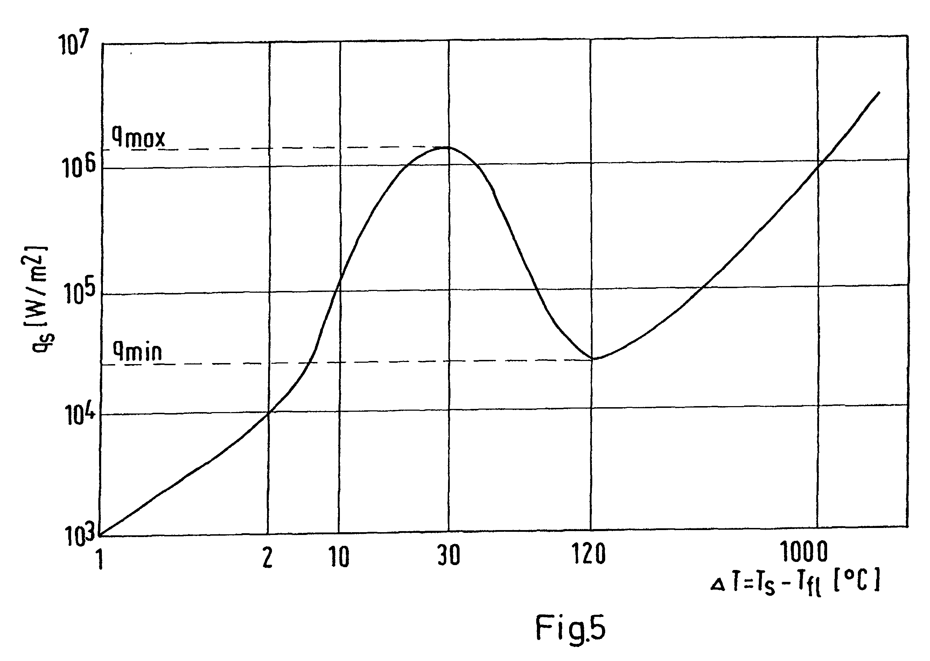

Das Diagramm der Figur 5 zeigt die typische Siedekurve für Wasser bei einer Atmosphäre. Es ist der Oberflächenwärmefluß $q_s$ in Watt pro Quadratmeter gegen die Temperaturdifferenz $\Delta T$ in Grad Celsius aufgetragen. Diese Temperaturdifferenz ergibt sich aus der aktuellen Temperatur minus der Temperatur der flüssigen Phase in Grad Celsius. Bei Wasser ist $T_{fl}$ = 100°C. Die schwarze Kurve, die Siedekurve für Wasser, zeigt ein erstes Maximum bei etwa $T_s$ = 130°C. Von da an nimmt die Wärmemenge des Wasserdampfes bei steigender Temperatur, welche zum Beispiel beim Überhitzen auf den Wasserdampf übertragen werden kann, allmählich ab bis zu einem miminalen Wert $q_{min}$ bei etwa 220°C.

Wünscht man bei der Vorrichtung zum Beispiel der Figur 1 einen Wärmefluß im Heizkörper 6 entgegen der Richtung der Pfeile 1 und 10, d.h. von unten nach oben, dann muß sich eine Temperaturdifferenz zwischen dem abstromigen Endbereich 17 des Heizkörpers 6 und seiner warmen Oberfläche 4 einstellen. Genau das gelingt dadurch, daß man die Temperatur der warmen Oberfläche auf einen Bereich zwischen 130°C und 140°C einstellt, wenn man Wasserstoffperoxid bzw. ein Gemisch Luft und Wasserstoffperoxid verdampfen und überhitzen will. Bei der Verdampfung wird $q_{max}$ übertragen, und beim Überhitzen wird die vergleichbare Wärmemenge um eine Größenordnung kleiner gehalten, so daß der gewünschte Wärmefluß entgegen der Strömungsrichtung der zu überhitzenden Dämpfe sich bildet und aufrechterhalten wird.

Bei einem praktischen Ausführungsbeispiel hat man 100 g $H_2O_2$ pro 1 kg Luft verwendet und dieses Gemisch aus Luft und Flüssigkeit verdampft und danach überhitzt. Gemäß dem Pfeil 1 der Figur 1 wurde die Heißluft bei 150°C zugepumpt und das Wasserstoffperoxid durch die Düse 3 in Nebel versprüht.

Im abstromigen Endbereich 17 des Heizkörpers 6 erreichte man eine Temperatur von etwa 214°C. Mit diesen Werten erreichte man im Sammeltrichter 9 ein Gemisch aus Luft und überhitztem Dampf, welches sich auf dem Weg durch die Ableitung 19 nach unten in Richtung des Pfeiles 10 bis zur Sammelflasche nicht kondensiert. In der Flasche selbst ist eine Kondensation zulässig aber nicht notwendig.

**Patentansprüche**

1. Verfahren zum Verdampfen und Überhitzen eines Sterilisierungsmittels, bei welchem das Sterilisierungsmittel mit Hilfe einer warmen Oberfläche (4) auf eine erste Temperatur gebracht und verdampft und der Dampf nachfolgend durch Heizelemente (8) auf eine zweite, höhere Temperatur gebracht und überhitzt wird, **dadurch gekennzeichnet, daß**

  a) das Sterilisierungsmittel auf die Wärmeoberfläche (4) aufgesprüht wird, die auf eine erste Temperatur erwärmt wird,
  b) welche kleiner ist als die Oberflächentemperatur, bei der das Filmsieden beginnt,
  c) die erste Temperatur der warmen Oberfläche (4) mit einem Temperaturfühler 5 abgefühlt und in Signale zur Regelung der Heizelemente (8) für das Überhitzen umgewandelt wird und
  d) der Dampf des Sterilisierungsmittels im Gegenstrom derart erwärmt wird, daß die erste Temperatur durch die zweite, höhere Temperatur und durch einen dem Dampfstrom entgegengerichteten Wärmefluß im wesentlichen konstant gehalten wird.

2. Verfahren nach Anspruch 1, **dadurch gekennzeichnet, daß** das Sterilisierungsmittel Wasserstoffperoxid ($H_2O_2$) ist.

3. Verfahren nach Anspruch 1 oder 2, **dadurch gekennzeichnet, daß** die erste Temperatur im Bereich von 100°C bis 150°C, vorzugsweise von 120°C bis 140°C liegt.

4. Verfahren nach einem der Ansprüche 1 bis 3, **dadurch gekennzeichnet, daß** ein gasförmiges Trägermedium in den Sprühstrahl (20) des Sterilisierungsmittels eingeführt wird.

5. Verfahren nach einem der Ansprüche 1 bis 4, **da-**durch gekennzeichnet, daß** das gasförmige Trägermedium eine Temperatur im Bereich von 130°C bis 170°C, vorzugsweise von 120°C bis 160°C und besonders bevorzugt von etwa 150°C hat.

6. Vorrichtung zum Verdampfen und Überhitzen eines Sterilisierungsmittels, bei welchem das Sterilisierungsmittel mit Hilfe einer warmen Oberfläche (4) auf eine erste Temperatur gebracht und verdampft wird und der Dampf nachfolgend durch Heizelemente (8) auf eine zweite, höhere Temperatur gebracht und überhitzt wird, mit einer Verdampfungskammer (2) und einem dieser nachgeschalteten Heizkörper (6) mit Überhitzungskanälen (7) und Heizelementen (8), **dadurch gekennzeichnet, daß**

  a) die warme Oberfläche (4) die aufstromige Oberfläche des Heizkörpers (6) ist,
  b) an welcher die Verdampfungskammer (2) direkt angebaut ist,
  c) In der Verdampfungskammer (2) gegenüber der warmen Oberfläche (4) eine mit einer nach außerhalb führenden Zuleitung (12) verbundene Düse (3) angebracht ist,
  d) von der aufstromigen Oberfläche (4) zu der abstromigen Endfläche (16) des Heizkörpers (6) offene, durchlaufende Kanäle (7) vorgesehen sind und daß
  e) die Heizelemente (8) im abstromigen Endbereich (17) des Heizkörpers (6) untergebracht sind, und
  f) ein Temperaturfühler (5) ein der ersten Temperatur entsprechendes Signal erzeugt, mit dem die Heizelemente (8) geregelt werden.

7. Vorrichtung nach Anspruch 6, **dadurch gekennzeichnet, daß** die Verdampfungskammer (2) mit einer Pumpe oder einem Gebläse für gasförmiges Trägermaterial verbunden ist und sich zur warmen Oberfläche (4) hin erweitert.

8. Vorrichtung nach Anspruch 6 oder 7, **dadurch gekennzeichnet, daß** der Heizkörper (6) aus einem Material mit gutem Wärmeleitungskoeffizienten gebildet ist.

9. Vorrichtung nach einem der Ansprüche 6 bis 8, **dadurch gekennzeichnet, daß** der Heizkörper (6) außen mit einer wärmeisolierenden Schicht versehen ist.

10. Vorrichtung nach einem der Ansprüche 6 bis 9, **dadurch gekennzeichnet, daß** der Heizkörper (6) ein massiver, zylinderförmiger Metallblock ist, die aufstromige Oberfläche (4) und die abstromige Endfläche (16) eben und mit Löchern (15) versehen sind, an welche sich gerade, parallele Kanäle (7) anschließen, und daß die aufstromig angebrachte Verdampfungskammer (2) und der abstromig angebrachte Sammeltrichter (9) zu den ebenen (4, 16) des Heizkörpers (6) sich erweiternde Trichter sind.

**Claims**

1. A method for vaporising and super-heating a sterilising agent, wherein the sterilising agent is brought to a first temperature and vaporised with the aid of a heated surface (4), and the vapour is subsequently brought to a second, higher temperature and super-heated by means of heating elements (8), **characterised in that**

   a) the sterilising agent is sprayed onto the heated surface (4), which is heated to a first temperature,

   b) which is lower than the surface temperature at which film boiling begins,

   c) the first temperature of the heated surface (4) is sensed with the temperature sensor (5) and converted into signals for controlling the heating elements (8) for super-heating, and

   d) the sterilising agent vapour is heated in the counter flow such that the first temperature is kept substantially constant by means of the second, higher temperature and by means of a flow of heat directed in the opposite direction to the flow of vapour.

2. Method according to claim 1, **characterised in that** the sterilising agent is hydrogen peroxide ($H_2O_2$).

3. Method according to claim 1 or 2, **characterised in that** the first temperature is in the range of 100°C to 150°C, preferably from 120°C to 140°C.

4. Method according to one of claims 1 to 3, **characterised in that** a gaseous carrier medium is introduced into the spray stream (20) of sterilising agent.

5. Method according to one of claims 1 to 4, **characterised in that** the gaseous carrier medium has a temperature in the range of 130°C to 170°C, preferably of 120° C to 160°C, and particularly preferably of approximately 150°C.

6. A device for vaporising and super-heating a sterilising agent, in which the sterilising agent is brought to a first temperature and vaporised with the aid of a heated surface (4), and the vapour is subsequently brought to a second, higher temperature and super-heated by means of heating elements (8), having a vaporising chamber (2) and a heater (6), super-heating channels (7) and heating elements (8) connected after said vaporising chamber, **characterised in that**

   a) the heated surface (4) is the up-stream surface of the heater (6),

   b) onto which the vaporising chamber (2) is directly mounted,

   c) a nozzle (30 connected to a supply line (12) leading to the exterior is fitted in the vaporising chamber (2), opposite the heated surface (4),

   d) open, continuous channels (7) are provided from the up-stream surface (40 to the down-stream end surface (16) of the heater (6), and that

   e) the heating elements (8) are housed in the down-stream end area (17) of the heater (6), and

   f) a temperature sensor (5) generates a signal corresponding to the first temperature by means of which the heating elements (8) are controlled.

7. Device according to claim 6, **characterised in that** the vaporising chamber (2) is connected to a pump or a blower for gaseous carrier material, and widens out towards the heated surface (4).

8. Device according to claim 6 or 7, **characterised in that** the heater (6) is formed from a material with a good thermal conduction coefficient.

9. Device according to one of claims 6 to 8, **characterised in that** the heater (6) is provided externally with a heat insulating layer.

10. Device according to one of claims 6 to 9, **characterised in that** the heater (6) is a solid, cylindrical block of metal, the up-stream surface (4) and the down-stream end surface (16) are planar and provided with holes (15), to which straight, parallel channels (7) are connected, and that the vaporising chamber (2) connected up-stream and the collecting funnel (9) connected down-stream are funnels widening out towards the planes (4, 16) of the heater (6).

**Revendications**

1. Procédé pour évaporer et surchauffer un agent stérilisant, dans lequel l'agent stérilisant est, à l'aide d'une surface chaude (4), chauffé à une première température et est évaporé, et la vapeur est ensuite, grâce à des éléments chauffants (8), portée à une deuxième température, plus élevée, et est surchauffée, **caractérisé en ce que** :

a) l'agent stérilisant est pulvérisé sur la surface chauffante (4), qui est chauffée à une première température,

b) laquelle est inférieure à la température superficielle à laquelle commence l'ébullition du film,

c) la première température de la surface chaude (4) est sondée à l'aide d'un capteur de température (5) et est convertie en des signaux destinés à la régulation des éléments chauffants (8) servant à la surchauffe, et

d) la vapeur de l'agent stérilisant est chauffée à contre-courant de façon que la première température soit maintenue essentiellement constante grâce à la deuxième température, plus élevée, et grâce à un flux thermique dirigé dans le sens inverse du courant de vapeur.

2. Procédé selon la revendication 1, **caractérisé en ce que** l'agent stérilisant est le peroxyde d'hydrogène ($H_2O_2$).

3. Procédé selon la revendication 1 ou 2, **caractérisé en ce que** la première température est comprise dans la plage de 100 à 150°C et de préférence de 120 à 140°C.

4. Procédé selon l'une des revendications 1 à 3, **caractérisé en ce qu'**un fluide porteur gazeux est introduit dans le jet de pulvérisation (20) de l'agent stérilisant

5. Procédé selon l'une des revendications 1 à 4, **caractérisé en ce que** le fluide porteur gazeux a une température comprise dans la plage de 130 à 170°C, de préférence de 120 à 160°C et plus particulièrement d'environ 150°C.

6. Appareillage pour évaporer et surchauffer un agent stérilisant, dans lequel l'agent stérilisant est, à l'aide d'une surface chaude (4), porté à une première température et évaporé, et la vapeur est ensuite, à l'aide d'éléments chauffants (8), portée à une deuxième température, plus élevée, et est surchauffée, comportant une chambre d'évaporation (2) et un corps chauffant (6), monté en aval de cette chambre, comportant des canaux de surchauffe (7) et des éléments chauffants (8), **caractérisé en ce que**

a) la surface chaude (4) est la surface côté amont du corps chauffant (6),

b) à laquelle est directement rapportée la chambre d'évaporation (2),

c) dans la chambre d'évaporation (2), une buse (3), qui communique avec une conduite d'amenée (12) menant vers l'extérieur, est disposée en regard de la surface chaude (4),

d) des canaux continus (7) sont prévus, qui sont ouverts de la surface côté amont (4) vers la face terminale (16) côté aval du corps chauffant (6), et

e) les éléments chauffants (8) sont disposés dans la zone terminale (17) côté aval du corps chauffant (6), et

(f) un capteur de température (5) produit un signal, correspondant à la première température et à l'aide duquel sont régulés les éléments chauffants (8).

7. Appareillage selon la revendication 6, **caractérisé en ce que** la chambre d'évaporation (2) communique avec une pompe ou avec une soufflante pour le matériau support gazeux, et s'élargit vers la surface chaude (4).

8. Appareillage selon la revendication 6 ou 7, **caractérisé en ce que** le corps chauffant (6) est constitué d'un matériau ayant un bon coefficient de conductibilité thermique.

9. Appareillage selon l'une des revendications 6 à 8, **caractérisé en ce que** le corps chauffant est, sur sa surface extérieure, pourvu d'une couche calorifuge.

10. Appareillage selon l'une des revendications 6 à 9, **caractérisé en ce que** le corps chauffant (6) est un bloc métallique cylindrique massif, la surface amont (4) et la surface terminale aval (16) sont planes et sont pourvues de trous (15), auxquels se raccordent des canaux rectilignes et parallèles (7) et **en ce que** la chambre d'évaporation (2), rapportée côté amont, et l'entonnoir collecteur (9), rapporté côté aval, sont des entonnoirs qui s'élargissent vers les plans (4, 16) du corps chauffant (6).

Fig.1

Fig.2

Fig.4

Fig.3

Fig.5

EP 0 956 054 B1